(19) **Europäisches Patentamt**

**European Patent Office**

**Office européen des brevets**

(11) **EP 1 621 215 A2**

(12) **EUROPEAN PATENT APPLICATION**

(43) Date of publication:
01.02.2006 Bulletin 2006/05

(51) Int Cl.:
*A61L 9/12* (2006.01)  *A61L 9/14* (2006.01)
*A61L 2/18* (2006.01)  *A61L 2/22* (2006.01)
*A61L 2/23* (2006.01)  *C08K 3/00* (2006.01)
*F25D 17/04* (2006.01)

(21) Application number: 05015572.0

(22) Date of filing: 18.07.2005

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: 23.07.2004 IT AP20040011

(71) Applicant: **Aria S.r.l.**
**60044 Fabriano (Ancona) (IT)**

(72) Inventor: **Pellegrini, Alfredo**
**60044 Fabriano**
**(Ancona) (IT)**

(74) Representative: **Reniero, Cirillo Silvano**
**c/o Dr.Reniero & Associati S.r.l.**
**Via D. Manin, 5**
**37122 Verona (IT)**

(54) **Sanitization process by using bacteriostatic or bactericidal products derived from vegetables**

(57) A sanitization process for obtaining a bacteriostatic or bactericidal action in an enclosed volume of space, comprising a stage of exposure of at least one bacteriostatic or bactericidal product obtained from at least one biologically grown vegetable in an amount ranging from 0.01 % v/v to 3% v/v with respect to said volume of space.

EP 1 621 215 A2

**Description**

The present invention relates to a process for making bacteriostatic or bactericidal objects, e. g. household appliances, in particular fridges, dishwashers and toys.

Living environments are generally affected by bacteria of various kinds. The immunity system in adults is quite efficient in preventing bacterium growth in the body. The same might not apply with childrens who are more liable to suffer from the consequences of exposure to environmental bacteria, such as those inevitably present in food, household appliances, toys and the like.

Bacteria can rapidly grow in high numbers, particularly in foodstuff preserved, e. g. in the fridge, so that the chances that a child living at home comes into contact with contaminated food or other objects are high and that might result in a development of an infectious disease. Food is kept sometimes in the fridge for relatively long time at a few degrees above 0C°, which is an ideal temperature for the growth of specific bacteria.

It has also been suggested to manufacture antibacterial fridges made of antibacterial plastics materials, i. e. plastics materials including one or more additives, such as silver ions. Unfortunately, in normal working conditions the antibacterial activity of said plastics materials is neutralized by a liquid film which is inevitably formed on the internal surfaces of the fridge owing to humidity.

It has also been suggested to provide an air-tight space arranged to receive food to be kept safe from bacteria inside a fridge where vacuum is created, e. g. by means of a suitable pump. This solution, besides being quite expensive, requires repeated upkeeping of the vacuum by the user.

Quite favourable conditions for rapid growth of bacteria are also to be found in dishwashers where dishes to be washed are charged in a succession of batches even at a distance of days before actual washing takes place, whereby bacteria can freely proliferate in most favourable conditions.

In the food industry the problem of slowing down the growth rate of bacteria is generally solved by adding conserving substances to foodstuff which are meant also to develop a desired antibacterial function although the conserving substances in general are not a warrant against toxicity. At home and in working facilities air conditioning systems are sometimes provided with bacteriostatic filters and/or UV-lamp apparatus or other sanitization appliances in order to sanitate the air flowing through the air conditioning systems. In practice, such air conditioning systems are quite expensive and in the medium run their bacteriostatic efficiency becomes drastically reduced.

Another long felt need is to make available antibacterial, and thus safer, toys. Toys, in fact, normally come into contact with bacterium contaminated surfaces and objects, such as floors, carpets, food, etc.. Moreover, in some cases toys, often manufactured in the Far East, are made even of toxic materials. Of course, children bring their toys to their mouth, and thus the exposure to bacteria is quite direct and poisoning.

An object of the present invention is to provide a sanitization process for obtaining bacteriologically sterile close environments or enclosures and surfaces of objects, such as toys, fridges, dishwashers, and the like.

Another object of the present invention is to provide specific bacteriostatic or bactericidal substances to be used in a sanitization process according to the invention.

Another object of the present invention is to provide objects having bacteriostatic or bactericidal surfaces.

A further object of the present invention is to provide objects having bacteriostatic or bactericidal surfaces which can be obtained at competitive prices.

According to a first aspect of the present invention there is provided a sanitization process for obtaining a bacteriostatic or bactericidal action in an enclosed volume of space, said process comprising the exposure of at least one antibacterial product obtained from at least one biologically grown vegetable in an amount ranging from 0.01 % v/v to 3% v/v, preferably from 0.05% v/v and 1.0% v/v with respect to said volume of space.

The expressions "enclosed volume of space" and "biologically grown vegetable" as used in the present description and in the claims attached to the description are to be understood as indicating, respectively, a volume of space fully surrounded, i. e. enclosed, by one or more solid surfaces, although being accessible through an opening and closing door means, and a vegetable cultivated in a conventional biological way, i. e. without making use of synthetic manures, herbicides or antiparasitic products.

According to another aspect of the present invention there is provided a sanitization process for obtaining a bacteriostatic or bactericidal objects or object surfaces made of thermoplastics material, said process comprising the inclusion in said material of at least one bacteriostatic or bactericidal product obtained from at least one biologically grown vegetable in an amount ranging from 5 to 25% w/w with respect to the amount of said thermoplastic material.

Advantageously, a biologically grown vegetable that can be used in a process in accordance with the present invention is selected from the group comprising: fir, yarrow, laurel, anise, orange, artemisia, basil, bergamot, birch, camomile, camphor, cinnamon, cypress, citronella, eucalyptus, fennel, clove, jasmine, juniper, geranium, incense, lavender, lemon, marjoram, mandarin, balm-mint, mint, musk, nutmeg, origan, mugo pine, silvester pine, rose, rosemary, sage, tea, thyme, valerian, vanilla, violet and ginger.

Further features and advantages of a sanitization process according to the present invention will better appear from the following detailed description of presently preferred Examples of carrying out the invention.

It has been found that antibacterial substances obtained from vegetables (plants) selected from the group comprising fir, yarrow, laurel, anise, orange, artemisia, basil, bergamot, birch, camomile, camphor, cinnamon, cypress, citronella, eucalyptus, fennel, clove, jasmine, juniper, geranium, incense, lavender, lemon, marjoram, mandarin, balm-mint, mint, musk, nutmeg, origan, mugo pine, silvester pine, rose, rosemary, sage, tea, thyme, valerian, vanilla, violet and ginger, have desired bacteriostatic or bactericidal properties especially in connection with sterilization of enclosed spaces and the manufacture of objects, such as household appliances (fridges, larders, dishwashers, etcetera), toys, covered dishes or the like, having long lasting sterilization or sanitization properties against bacteria in general.

According to the present invention the above-mentioned antibacterial substances, are preferably in a liquid state, and are relatively easily vaporizable, whereby they can develop from a container-dispenser to reach surfaces of objects, e. g. the inner wall or walls of an enclosed volume of space, or can be applied to them by means of a brush or the like or directly sprayed onto them, thereby developing a sanitization action against bacteria both at the surfaces to which it is applied and in the nearby environment.

The sanitization action developed by such antibacterial substances is effective in the sense of exerting a destroying action on bacteria and/or a slowing down action on bacteria growth rate.

Even if an object surface exposed to vapours of an antibacterial substance according to the invention is affected by a liquid film, e. g. formed onto it by condensation phenomena, the sanitization effect is uninterruptedly assured owing to the continuous development of antibacterial substance vapour that replace any previous vapour washed away by condensation water.

Tests have been conducted to demonstrate the bacterium proliferation slow rate on enclosed volume of spaces exposed to a predetermined amount of one or more of above-mentioned antibacterial substances.

In the following description of tests 1 to 3, antibacterial substance concentrations are concentrations by volume evaluated with respect to the bacterium growth soil.

TEST 1

Evaluation of bacteriostatic and bactericidal action of antibacterial substances added to a growth soil versus *Escherichia Coli* bacterium

Bacterium batches were grown on Petri plates filled with a growth soil containing nutritive compounds and agar. The soil comprised solid soil and a physiologic solution generally used for antibacterial tests, as it is well known to a skilled person in the art.

The antibacterial substances used in the test were:

A: clove oil;
B: silvester pine oil;
C: lavender oil; and
D: a mixture of clove and silvester pine oils.

Initially, the antibacterial substances were added to the bacterium growth soil at a concentration of 1% v/v (concentration by volume with respect to the growth soil) in order to evaluate their antibacterial properties. The growth soil was then stirred to obtain a uniform distribution of the antibacterial substances and incubated for 24 hours at 37°C in a thermostated chamber.

The test results are summarized in the following Table 1 in which the number of bacteria found at the end of the test is indicated by the Colony forming unit (CFU).

Table 1 CFU after 24 hours of incubation at 37°C of a growth soil added with 1% of antibacterial substances

| Antibacterial substance added to the growth soil | CFU after 24 hours at 37°C |
| --- | --- |
| Absent | 270 |
| A: clove oil | 64 |
| B: silvester pine oil | 0 |
| C: lavender oil | 0 |
| D: mixture of clove and silvester pine oil | 220 |

Substances B and C at a concentration of 1% showed a bactericidal action, whereas substances A and D still at a concentration of 1% showed a bacteriostatic effect, i. e. substances B and C did sterilize the growth soil, whereas substances A and D caused a slowing down in the bacterium growth rate.

Substances B and C, which showed better antibacterial properties with respect to substances A and D, were then added to a growth soil at a concentration of 0.1% v/v (concentration by volume with respect to the growth soil).

The results are shown in the following Table 2, in which the number of bacteria as counted at the end of the test is indicated by CFU values.

Table 2 CFU values after 24 hours of incubation at 37°C of a growth soil to which an amount of 0.1% v/v of antibacterial substances was added

| Antibacterial substance added to the growth soil | CFU after 24 hours at 37°C |
|---|---|
| Absent | 300 |
| B: silvester pine oil | 290 |
| C: lavender oil | 0 |

Antibacterial substance C, i. e. lavender oil, showed a bactericidal action at a concentration of 0.1% v/v.

Substance C was then further tested at concentrations of 0.1 % v/v and 0.05% v/v.

The results are shown in the following Table 3.

Table 3 Number of bacteria after 24 hours of incubation at 37°C in a growth soil to which 0.1 % v/v and 0.05% v/v of substance C were added

| Antibacterial substance added to the growth soil | Number of bacteria (after 24 hours) | Colony diameter after 24 hours | Colony diameter after 48 hours |
|---|---|---|---|
| Absent | 47 | 5 mm | 6 mm |
| C: lavender oil at 0.1% v/v | 0 | 0 mm | 0 mm |
| C: lavender oil at 0.05% v/v | 4 | 2.5 mm | 4 mm |

Substance C at a concentration of 0.1% v/v showed a bactericidal properties, whereas substance C showed a bacteriostatic properties at a concentration of 0.05% v/v.

TEST 2

Substances B and C evaporation rate at room temperature

Substances B and C were inserted in a thermostated chamber at room temperature and the substance weight/surface ratio for both of them was evaluated. Both substances B and C, alone or in mixture with a gel, were controlled and the loss of weight versus time was recorded.

The resulting data of the test were interpolated so as to obtain two algorithms indicating, respectively, the evaporation rate of B and C.

To this end two different interpolation methods were used.

The algorithm adopted for the first interpolation method is:

$$p_0/p = a + b \sqrt{\min uti} \qquad (1)$$

| Antibacterial substance | parameter a | parameter b |
|---|---|---|
| B | 0.336093 | -0.0007833 |
| C | 0.354364 | -0.00008161 |

Table continued

| Antibacterial substance | parameter a | parameter b |
|---|---|---|
| Gel + oil B | 0.58377 | -0.00604 |
| Gel + oil C | 0.65788 | -0.009935 |

The algorithm adopted for the second interpolation method is:

$$p_0/p = (A \times T_{1/2})/(T_{1/2} + \min \textit{uti}) \qquad (2)$$

| Antibacterial substance | parameter A | parameter $T_{1/2}$ |
|---|---|---|
| B | 0.352217 | 854737 |
| C | 0.322321 | 46856 |
| Gel + oil B | 0.616238 | 1259 |
| Gel + oil C | 0.56782 | 1956 |

Parameters used in algorithms (1) and (2) have the following meaning:

p: initial weight of the antibacterial substances expressed in g;
$p_0$: weight of the antibacterial substances at time t, expressed in g;
a, b, A: correlation parameters; and
$T_{1/2}$: half-life time of the antibacterial substances.

The volatization rates of substances B and C were found to be extremely different from one another. Moreover, half-life times differ of about one order of magnitude from one another. The substance B has a volatization rate higher than substance C and thus the bacterium growth inhibiting properties of substance C is greater than that of substance B as the useful inhibiting life of substance C is much longer than that of substance B.

TEST 3

Evaluation at 37°C and up to 144 hours of bacteriostatic and bactericidal action of substance C versus *Escherichia Coli* bacterium

The substance C was distributed on a surface of area of 16 cm$^2$ and kept inside a volume of a space delimited by a container at a temperature of 21 °C. Two further plates each having a contact surface of 61 cm$^2$ and previously charged with growth soil contaminated with Escherichia Coli bacteria were inserted into such an enclosure or environment. The bacteria were exposed to substance C for 24 hours and the CFU (Colony forming Units) and the size of each bacterium colony were estimated in order to evaluate the antibacterial properties of substance C.
The test results are summarized in the following Table 4.

## Table 4

### CFU after 24 hours of incubation at 37°C of a growth soil to which an amount of 0.1% of antibacterial substance C was added

| Antibacterial substance | CFU after 24 hours at 37°C | | | | |
|---|---|---|---|---|---|
| | I Example | II Example | III Example | IV Example | V Example |
| absent | 281 | 338 | 325 | 321 | 13 |
| oil C | 330 | 308 | 313 | 315 | 11 |

| Antibacterial substance | CFU after 24 hours at 37°C | | | |
|---|---|---|---|---|
| | VI Example | VII Example | VIII Example | IX Example |
| absent | 12 | 61 | 60 | 80 |
| oil C | 8 | 60 | 88 | 75 |

These tests were somehow astray and in any case insufficient to evaluate the inhibition properties on the bacterium growth rate of substance C. In view of this, further tests were conducted up to 144 hours and at a growth temperature of 21°C.

The test results are summarized in the following Table 5.

Table 5 CFU after 48, 72 and 144 hours of incubation at 21°C of a growth soil to which an amount of 0.1 % of antibacterial substance C was added

| Antibacterial substance | CFU after 48 hours at 37°C | | Colony diameter after 48 hours |
|---|---|---|---|
| | X Example | XI Example | |
| absent | 35 | 47 | 1 mm |
| oil C | 16 | 28 | 0.6 mm |
| Antibacterial substance | CFU after 72 hours at 37°C | | Colony diameter after 72 hours |
| | XII Example | XIII Example | |
| absent | 43 | 40 | 3 mm |
| oil C | 20 | 20 | 2 mm |
| Antibacterial substance | CFU after 144 hours at 37°C | | Colony diameter after 144 hours |
| | XIV Example | XV Example | |
| absent | 35 | 41 | 5.3 mm |
| oil C | 12 | 39 | 3.2 mm |

The results can be interpolated by means of the algorithm

$$mm= a + b/hours \qquad (3)$$

where:

mm: millimetre diameter of the colony;
a, b: numeric parameters

Table 5 Parameter values of algorithm (3)

| Antibacterial substance | parameter a | parameter b | $r^2$ |
|---|---|---|---|
| Absent | 7 | -288 | 1 |
| C | 4.26667 | -172.8 | 0.99 |

The bacterium growth slowing down at room temperature owing to the present of substance C is valuable on the basis of CFU size. The interpolation algorithm (3) allows to foresee the rate of such growth.

TEST 4

Evaluation of bacteriostatic and bactericide action of substance C versus *Escherichia Coli* bacterium at fridge temperatures

Predetermined amounts of substance C, maintained in different containers (made of plastics, tinfoil and glass, respectively) were inserted into the enclosure or environment delimited in non ventilated fridge in order to evaluate weight loss values due to evaporation of oil C, all the amounts being brought and kept at temperatures of 4°C, 7°C, 10°C, these temperatures being those usually to be found at various levels inside a fridge.
The test results are summarized in the following Table 6.

Table 6 Evaporation rate of substance C inside a fridge

| Material | Evaporation Rate at 4°C g/cm$^2$*min | Evaporation Rate at 7°C g/cm2*min | Evaporation Rate at 10°C g/cm2*min |
|---|---|---|---|
| Plastics | 11.1 | 6.8*3.8E-7 | 4.6*1.3E-7 |
| Tinfoil | 10.5*8.5E-7 | 7.1*1.0E-6 | 4.1*6.3E-7 |
| Glass | 11.6*1.1E-7 | 8.3*9.8E-8 | 5.1*9.7E-8 |

The above test results show that antibacterial substances according to the invention inside a fridge have an evaporation rate quite suitable to act as antibacterial agents even at temperature range from about 4 to 10°C.
In the above Examples and tests, the same useful concentration in percentage (from about 0.01 to 3.0) of antibacterial substances referred to the growth soil was used when referred to the volume of space in which the tests where conducted.
According to the present invention the above mentioned antibacterial substances, preferably in a liquid state, were blended, preferably mechanically blended, with a thermoplastics material such as a polymer, e. g. rigid rubber Marfran (soft bead rubber), Doki Polystyrene 485, Vamplen 0024 Vo Black A (black bead polypropylene with self-extinguishing class V0), Agrilene® 00NR2FR (black polypropylene), all of which are sold by the applicant. The obtained mixture was loaded into an injection molding press and molded objects (e. g. discs, small toys, cutlery articles) were obtained.
The antibacterial substances, in a liquid state, can be added to a thermoplastic material in a concentration up to 15% w/w (concentration by weight with respect to the amount of the thermoplastic material), preferably up to 10% w/w, more preferably about 8% w/w. Using a concentration higher than 15%w/w the molding operation becomes more difficult to be carried out. With percentages ≤ 15%w/w of antibacterial substances antibacterial properties of molded objects incorporating such antibacterial substances have been tested to persist beyond 6 months.
It was found that antibacterial useful life of the antibacterial substances incorporated in molded objects was higher when antibacterial surfaces in a solid state (powder) were added to plastics materials. Thus, antibacterial substances are added to plastics materials up to 25% w/w, preferably from 15% to 20% w/w, to obtain molded objects with an antibacterial useful life of at least about 8-10 months.

Tests were conducted by Ecoricerche S.r.l. of Capua - Italy - on behalf of the applicant to evaluate the antibacterial properties of molded object made of mixtures comprising antibacterial substances and thermoplastic materials.

TEST 5

Contact test of antibacterial properties of objects obtained by molding a mixture comprising antibacterial substance and thermoplastic materials

5 samples of a polymer incorporating antibacterial substances (each marked by "sample with additive") and 1 sample of non-treated polymer (marked "neutral") were subjected to contact tests, i. e. the samples are brought into contact with a batch of bacteria. All 6 samples were contaminated in a controlled environment (a laminar-flow hood model Bench Flow - flow rate 300 mc/h, rate 0.45 m/s) with a known amount of CFU of *Escherichia Coli*, used *Escherichia Coli* strain was a controlled and certified strain supplied by Sieroterapico Soc. Coop a R. L. Diagnostici of Milan - Italy.

The test samples had size 5x5x0.3 cm and weight 6.7 $\pm$ 0.3 g.

Contamination was carried out by inoculating onto the surface of each sample about 100 $\mu$l of a revitalized suspension of the *Escherichia Coli* strain referred to above (Code 76/31 ex ATCC 8739) corresponding to 1700 CFU, to which a surfactant agent (Tween 80) was added by means a sterile loop to improve the bacterium spreading uniformity throughout the surface area of the polymeric samples.

The samples were located onto a Petri plate 90 cm in diameter so as to keep unaltered the amount of water in the inoculated suspension.

At intervals of 2, 4, 8, 16 or 32 hours after inoculation of the suspension, the contaminated polymeric samples were cleaned by means of sterile buffers, treated (recovery) with a Maximum Recovery Diluent peptone solution (sold by Biofile Italiana s.r.l. of Milan), incubated for 24 hours at a temperature of 44°C on m-Faecal Coliform Agar (sold by Biofile Italiana s.r.l.).

The test results are summarized in the following Table 7.

Table 7 CFU evaluated after 34 hours of incubation

| Contact time (hours) of *E. Coli* with sample before recovery and incubation | CFU reckoned after 24 hours of incubation at 44°C |
|---|---|
| 0 (with no antibacterial substance) | 1700 |
| 2 | <50 |
| 4 | <10 |
| 8 | <10 |
| 16 | <10 |
| 32 | <10 |

TEST 6

Aereodispersion test of antibacterial properties of molded objects obtained by molding a mixture comprising antibacterial substance and plastics materials

To polymer samples (discs) each having a diameter of 6 cm an antibacterial substance of TEST 5 was added and the samples were then placed in a 6-litres laboratory glass desiccator. Neutral samples were contaminated in the same manner as specified in TEST 5 and loaded into the desiccator. The polymer samples were covered with sterile absorbing paper to maintain humidity thereon for a relative long time, the inner volume of the desiccator being greater than that of the Petri plate.

At intervals of 2, 4, 8, 16 or 32 hours from closing the desiccator the absorbing paper on each sample was recovered and incubated as described in TEST 5.

The test results are summarized in the following Table 8.

Table 8 UFC evaluated after 34 hours of incubation

| Exposure time (hours) of *E. Coli* on samples before recovery and incubation | UFC computed after 24 hours of incubation at 44°C |
|---|---|
| 0 (with no antibacterial substance) | 2100 |
| 2 | 150 |
| 4 | <50 |
| 8 | <10 |
| 16 | <10 |
| 32 | <10 |

The tests conducted showed excellent antibacterial properties of the above-mentioned antibacterial substances.

After a few months from incorporation of substances B and C, their bacterium growth inhibiting properties were still detectable in the objects incorporating them.

The process as above described is susceptible to numerous modifications and variations within the scope as defined by the claims.

**Claims**

1. A sanitization process for obtaining a bacteriostatic or bactericidal action in an enclosed volume of space, **characterized in that** it comprises a stage of exposure of at least one bacteriostatic or bactericidal product obtained from at least one biologically grown vegetable in an amount ranging from 0.01 % v/v to 3% v/v with respect to said volume of space.

2. A sanitization process as claimed in claim 1, **characterized in that** said at least one bacteriostatic or bactericidal product has a concentration by volume ranging from 0.05% v/v to 1.0% v/v with respect to said volume of space.

3. A sanitization process as claimed in claim 1 or 2, **characterized in that** said at least one bacteriostatic or bactericidal product has a concentration by volume of about 0.1% v/v with respect to said volume of space.

4. A sanitization process as claimed in any claim 1 to 3, **characterized in that** said biologically grown vegetable is selected from the group comprising: fir, yarrow, laurel, anise, orange, artemisia, basil, bergamot, birch, camomile, camphor, cinnamon, cypress, citronella, eucalyptus, fennel, clove, jasmine, juniper, geranium, incense, lavender, lemon, marjoram, mandarin, balm-mint, mint, musk, nutmeg, origan, mugo pine, silvester pine, rose, rosemary, sage, tea, thyme, vanilla, violet and ginger.

5. A sanitization process as claimed in any claim 1 to 4, **characterized in that** it comprises the stages:

   - loading said at least one bacteriostatic or bactericidal product into said enclosed volume of space; and
   - evaporation of said at least one bacteriostatic or bactericidal product.

6. A sanitization process as claimed in claim 5, **characterized in that** evaporation of said at least one bacteriostatic or bactericidal product takes place at a temperature ranging between -5°C and 40°C.

7. A sanitization process as claimed in any claim 1 to 6, **characterized in that** said volume of space is delimited inside a household appliance.

8. A sanitization process for obtaining a bacteriostatic or bactericidal objects or object surfaces made of at least one plastics material, **characterized in that** it comprises incorporation in said plastics material or materials of at least one bacteriostatic or bactericidal product obtained from at least one biologically grown vegetable in an amount ranging from 5 to 25% w/w with respect to the amount of said plastics material.

9. A sanitization process as claimed in claim 8, **characterized in that** said biologically grown vegetable is selected from the group comprising: fir, yarrow, laurel, anise, orange, artemisia, basil, bergamot, birch, camomile, camphor, cinnamon, cypress, citronella, eucalyptus, fennel, clove, jasmine, juniper, geranium, incense, lavender, lemon, marjoram, mandarin, balm-mint, mint, musk, nutmeg, origan, mugo pine, silvester pine, rose, rosemary, sage, tea, thyme, vanilla, violet and ginger.

10. A sanitization process as claimed in claim 8 or 9, **characterized in that** it comprises the stages of:

- adding said at least one bacteriostatic or bactericidal product to a predetermined amount of plastics beads;
- loading a batch of said plastics beads into a forming mold;
- molding said plastics beads in said forming mold to obtain at least one object provided with antibacterial properties.

11. A sanitization process as claimed in any claim 8 to 10, **characterized in that** said at least one bacteriostatic or bactericidal product is in a liquid form.

12. A sanitization process as claimed in claim 11, **characterized in that** the concentration of said at least one bacteriostatic or bactericidal product ranges from 1% to 15% w/w with respect to said plastics material.

13. A sanitization process as claimed in claim 11, **characterized in that** the concentration of said at least one bacteriostatic or bactericidal product ranges from 8% to 10% w/w with respect to said plastic material.

14. A sanitization process as claimed in any claim 8 to 10, **characterized in that** said at least one bacteriostatic or bactericidal product is in a solid form.

15. A sanitization process as claimed in claim 14, **characterized in that** said solid form comprises powder form.

16. A sanitization process as claimed in claim 14 or 15, **characterized in that** the concentration of said at least one bacteriostatic or bactericidal product ranges from 15% to 20% w/w with respect to said plastics material.

17. A sanitization process as claimed in any claim 8 to 16, **characterized in that** said at least one plastics material comprises at least one rubber material.

18. Object when obtained in accordance with a process as claimed in any claim 1 to 17.